# EUROPEAN PATENT APPLICATION

(11) **EP 0 677 293 A1**
(43) Date of publication of application: **18.10.1995**
(21) Application number: 94903094.4
(22) Date of filing: 28.12.1993
(51) Int. Cl.: A61K 31/44, C07D 213/22

(54) **PHARMACEUTICAL USE OF TERPYRIDINE DERIVATIVE**

(30) Priority: 28.12.1992 JP 348684/92
(71) Applicant: TAISHO PHARMACEUTICAL CO. LTD, Tokyo 171 (JP)
(72) Inventor: SHIRAI, Hirofusa, Chiisagata-gun, Nagano 386-04 (JP); HANABUSA, Kenji, Ueda-shi, Nagano 386 (JP); TAKAHASHI, Yuki, Taisho Pharmaceutical Co., Ltd., Tokyo 171 (JP); MIZOBE, Fumio, Taisho Pharmaceutical Co., Ltd., Tokyo 171 (JP); HANADA, Kazunori, Taisho Pharmaceutical Co., Ltd., Tokyo 171 (JP)
(74) Representative: VOSSIUS & PARTNER
(86) International application number: JP9301921
(87) International publication number: WO9414440

(57) **Abstract**

A terpyridine derivative represented by general formula (I) (wherein R represents hydrogen, (un)substituted phenyl, pyridyl, etc.), which has the effect of promoting the production of nerve growth factors or a neurotrophic factor activity. Hence it can be used as an ameliorant or remedy for the symptoms and diseases accompanying peripheral nerve degeneration which is idiopathic or caused by trauma, chemicals such as alcohol or anticancer drug, inflammation, or metabolism as observed in, for example, diabetes. In addition, it can be used as an ameliorant or remedy for the symptoms and diseases accompanying central nerve degeneration, such as senile dementia of Alzheimer type, cerebrovascular dementia, Down's syndrome, Parkinson's disease or Hantington's chorea, mental and motor function incompetences caused by cerebral ischemia, cerebral infarction, cerebral hemorrhage or head injury, spinal neuroparalysis, and so forth.

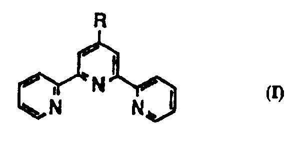

## Description

### Technical Field

The present invention relates to a pharmaceutical use of a terpyridine derivative which exerts a nerve growth factor (hereinafter abbreviated as NGF) synthesis-stimulating activity or a neurotrophic factor activity. In more detail, the present invention relates to a pharmaceutical composition comprising a terpyridine derivative as an effective ingredient, for improving or treating diseases caused by nerve degeneration and to a method for the improvement or treatment of diseases caused by nerve degeneration which comprises administering an effective dose of a terpyridine derivative to human.

### Background Art

Senile dementia of Alzheimer type has been increasing in these years. It is suggested that degeneration or cast-off of acetylcholinergic nerves, which are cerebral basal neurons, might be deeply involved in this type of senile dementia to cause dysmnesia and reduction in intellectual activities, see Witehorse et al., Science, 215, 1237 (1982).

It is reported that NGF not only prevent central acetylcholinergic nerves from degeneration or falling off due to breakage of nerve fibers but also improves maze-learning disturbance of aged rats and prevents atrophy of acetylcholinergic nerve cells, see Taku Shigeno et al., IGAKU-NO-AYUMI, 145, 579 (1986). These studies suggest that NGF would be effective for the treatment of Alzheimer type senile dementia.

It is also shown that NGF prevents death of hippocampus nerve cells in Monglian gerbil with cerebral ischemia and is thus considered to be effective for the treatment of cerebral apoplexy.

On the other hand, NGF exhibits an action for promoting recovery of peripheral nerves from damages; it has been revealed that NGF is effective also for the treatment of peripheral nerve disorders.

Many biological components other than NGF have been found to show an activity for maintaining survival and functions of nerve cells or to show an activity for repairing nerve degeneration. These biological components are collectively referred to as neurotrophic factors. Therefore, these neurotrophic factors are considered to be effective for the treatment of central nervous system disorders and peripheral nervous system disorders associated with degeneration of nerve cells.

The biological components termed neurotrophic factors are all proteins, including NGF. When proteins are applied as drugs for the treatment of central nervous system disorders, it is highly likely, in terms of their physical properties, that directly intraventricular administration of these proteins are required and hence, problems encounter for practical application. Therefore, it is desired to develop a low molecular drug having a neurotrophic activity or an activity for accelerating the production of a neurotrophic factor applicable in a simpler fashion.

### Disclosure of Invention

As a result of extensive investigations of a number of compounds to achieve the foregoing object, the present inventors have discovered that some terpyridine compounds are physiologically active and exhibit either the NGF synthesis-stimulating activity or the neurotrophic activity, or both of these activities. The present invention has thus been attained.

An object of the present invention is to provide a pharmaceutical composition comprising as an effective ingredient a terpyridine derivative represented by formula (I): 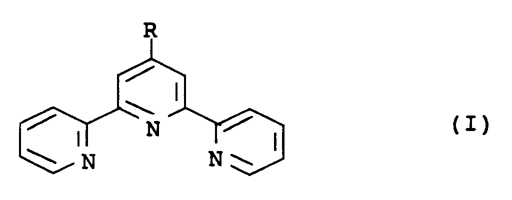
wherein R represents a hydrogen atom; an unsubstituted phenyl group; a phenyl group substituted with at least one substituent selected from the group consisting of an alkyl group, an alkoxy group, hydroxy group, nitro group, amino group, carboxy group, bromomethyl group, 2-hydroxyethoxy group, 2-acetoxyethoxy group and a halogen atom; pyridyl group; or a functional group represented by any one of (II)-1 through (II)-8 shown below: 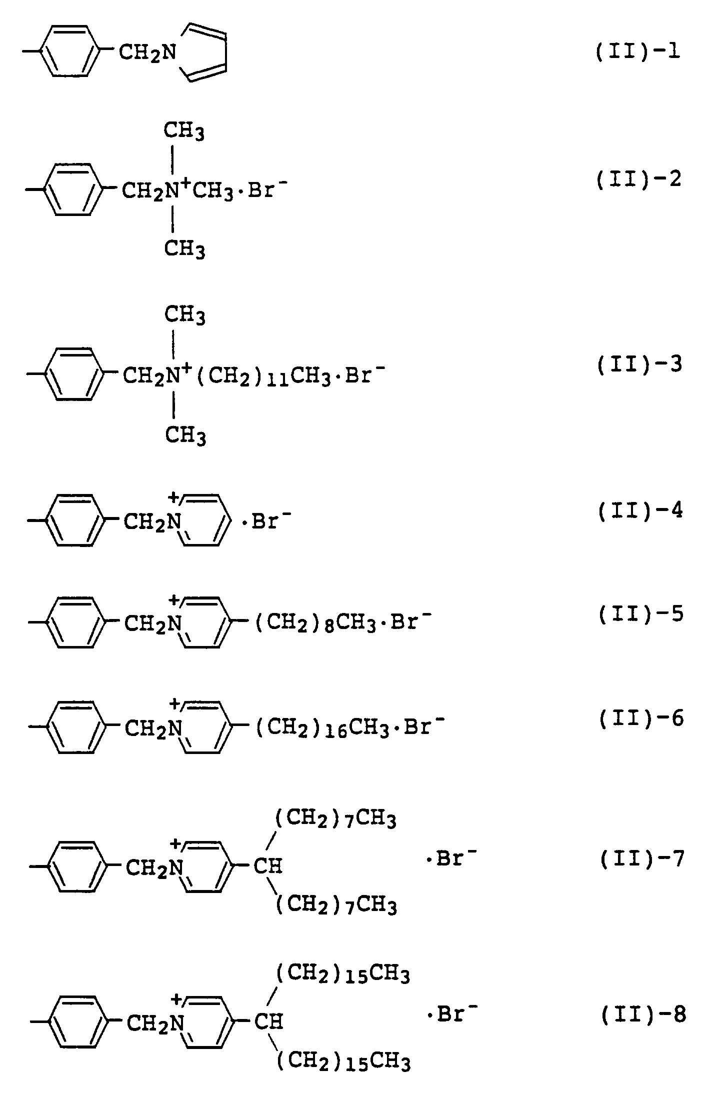
or a pharmaceutically acceptable salt thereof.

Another object of the present invention is to provide the aforesaid terpyridine derivative or a pharmaceutically acceptable salt thereof, for use as an effective ingredient in a pharmaceutical composition.

A further object of the present invention is to provide a use of the aforesaid terpyridine derivative or a pharmaceutically acceptable salt thereof, to prepare a pharmaceutical composition for improving or treating diseases caused by nerve degeneration.

A still further object of the present invention is to provide a method for improving or treating diseases caused by nerve degeneration which comprises administering to human an effective dose of the aforesaid terpyridine derivative or a pharmaceutically acceptable salt thereof.

### Best Mode for Carrying Out the Invention

In the terpyridine derivative represented by formula (I) where R is a phenyl group substituted with an alkyl group, examples of the alkyl group are those having 1 to 6 carbon atoms such as methyl, ethyl, propyl, isopropyl, butyl, t-butyl, pentyl and hexyl. Where R is a phenyl group substituted with an alkoxy group, examples of the alkoxy group are those having 1 to 6 carbon atoms such as methoxy, ethoxy, propoxy, isopropoxy, butoxy, pentoxy or hexyloxy. Where R is a phenyl group substituted with a halogen atom, examples of the halogen atom include fluorine, chlorine and bromine.

As the pharmaceutically acceptable salts of the terpyridine derivative, there are acid addition salts with mineral acids such as hydrochloric acid, sulfuric acid, phosphoric acid or nitric acid, or with organic acids such as citric acid, succinic acid, tartaric acid or methanesulfonic acid. There are also salts with alkali metals such as sodium or potassium, and with alkaline earth metals such as calcium.

The terpyridine derivatives of the present invention represented by formula (I) are partly novel but includes some known compounds. That is, the terpyridine derivatives of formula (I) include known compounds described in F.H. Case et al., J. Organic Chemistry, 26, 4415-4418 (1961) and W. Spahni et al., Helvechica Chemica Acta, 67, 450-454 (1984) and known compounds the inventor publicly reported at the Nippon Kagakukai (Japanese Chemical Society), The 61st Spring Annual Meeting, 1991.

The terpyridine derivatives of the present invention can be prepared, e.g., by the following processes.
i) The compounds represented by formula (I) wherein R represents an unsubstituted phenyl group, a phenyl group substituted with an alkyl group, an alkoxy group, hydroxy group, nitro group, 2-hydroxyethoxy group or a halogen atom, or a pyridyl group, can be prepared as follows.
   Firstly, 2-acetylpyridine is condensed with a compound shown by formula (III):

   R¹ - CHO (III)

   wherein R¹ represents an unsubstituted phenyl group, a phenyl group substituted with an alkyl group, an alkoxy group, hydroxy group, nitro group, 2-hydroxyethoxy group or a halogen atom, or a pyridyl group, in the presence of an alkali to give the compound shown by formula (IV): 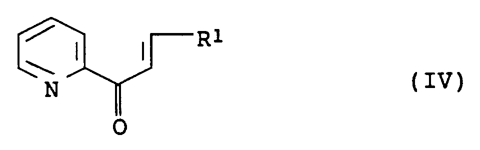 wherein R¹ has the same significance as described above.
Herein, examples of the alkali used in the condensation are potassium hydroxide, sodium hydroxide, sodium methoxide, potassium t-butoxide, potassium carbonate, sodium carbonate, sodium hydrogencarbonate, or potassium hydrogencarbonate. As a solvent for the reaction, methanol, ethanol, n-propanol, isopropanol or t-butanol may be used singly or in combination with water. The reaction temperature can be appropriately chosen from 0°C to the boiling point of a solvent used.
Then, the compound of formula (IV) is reacted with a compound represented by formula (V): 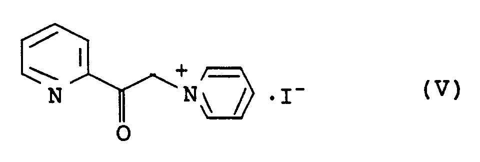 which can be prepared by the method of F. Krohnke et al., Chemische Berichte, 92, 22 (1959), in the presence of ammonium acetate to give the compound of the present invention. At this stage of the reaction, ammonium acetate is employed in a 1- to 10-fold molar amount based on the compound of formula (IV). As a solvent for the reaction, methanol, ethanol, n-propanol, isopropanol, t-butanol or acetic acid may be used. The reaction temperature can be appropriately chosen from room temperature to the boiling point of a solvent used.
ii) The compound of formula (I) wherein R is an amino-substituted phenyl group can be prepared by reducing the compound of formula (I) obtained by the above process, wherein R is a nitro-substituted phenyl group. The reduction can be performed in a conventional manner, e.g., by catalytic hydrogenation or reduction in ethanol using hydrazine and Raney nickel.
iii) The compound of formula (I) wherein R is a phenyl group substituted with one or two carboxy group(s) can be prepared by oxidizing the compound of formula (I) obtained by the above process, wherein R is a monomethyl-substituted or dimethyl-substituted phenyl group. The oxidization can be performed in a conventional manner, e.g., by oxidization using a permanganate.
iv) The compound of formula (I) wherein R is a phenyl group substituted with bromomethyl group can be prepared by brominating the compound of formula (I) obtained by the above process, wherein R is a methyl-substituted phenyl group. The bromination can be performed in a conventional manner, e.g., by reacting with N-bromosuccinimide in a halogen solvent.
v) The compound of formula (I) wherein R is any one of the functional groups shown by formula (II)-1 through (II)-8 can be prepared by animating or quaternizing the compound of formula (I) obtained by the above process, wherein R is a bromomethyl-substituted phenyl group, with the corresponding amine. As a solvent for the reaction, water, acetone, an alcohol (e.g., methanol or ethanol), diethyl ether, benzene or acetonitrile may be employed singly or as admixture thereof. The reaction temperature can be appropriately chosen from 0°C to the boiling point of a solvent used.
vi) The compound of formula (I) wherein R is a hydrogen atom can be prepared by the method of E.C. Constable et al., Inorganica Chimica Acta, 141, 201 (1988).

Representative examples of the terpyridine derivatives in accordance with the present invention are given in Table 1 below.

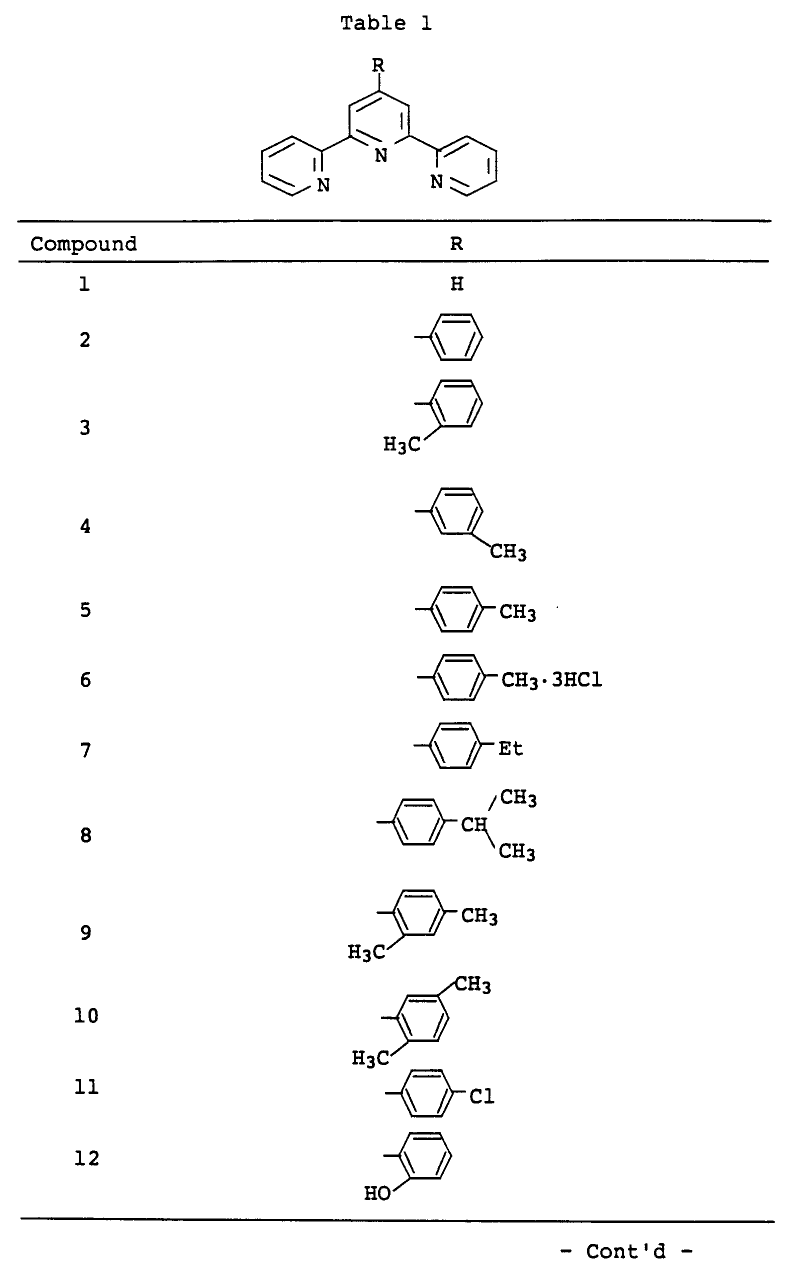

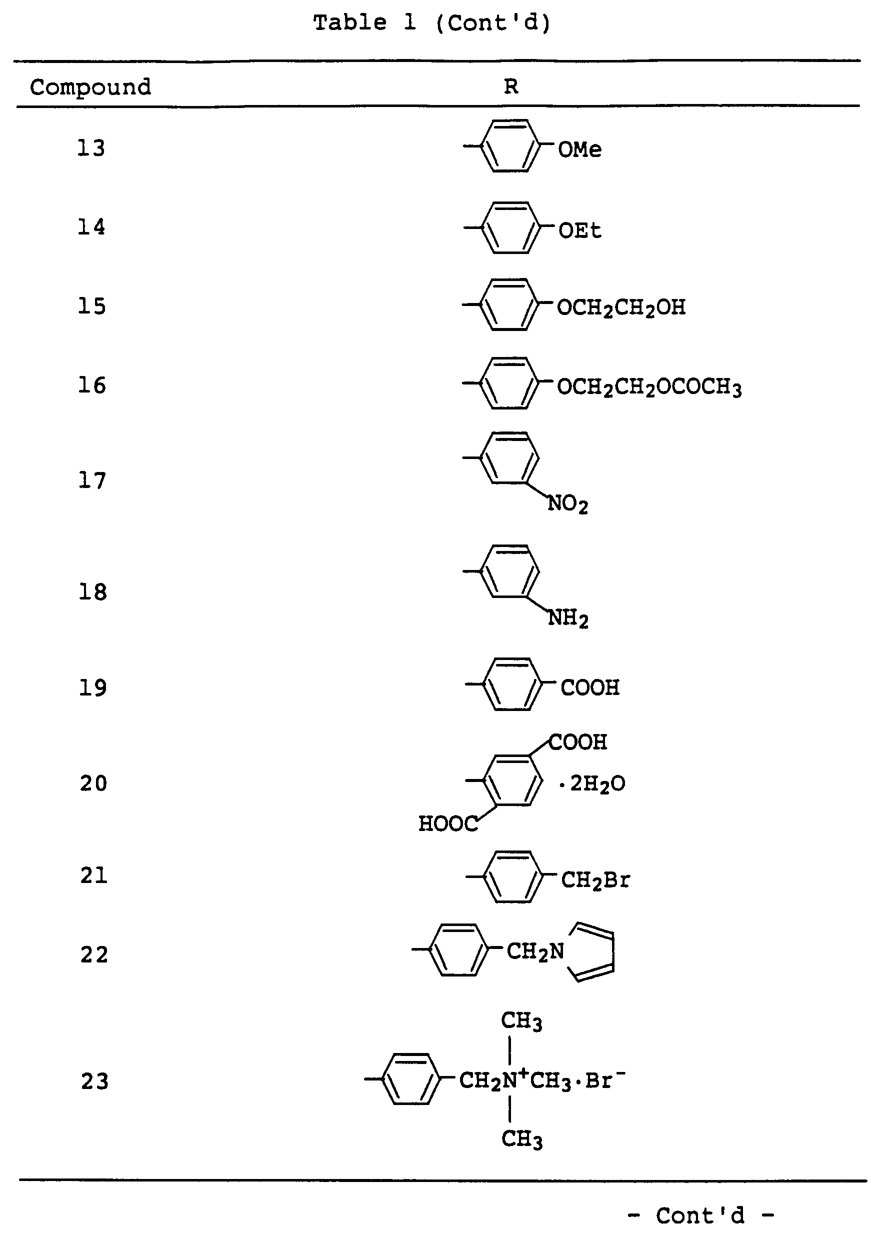

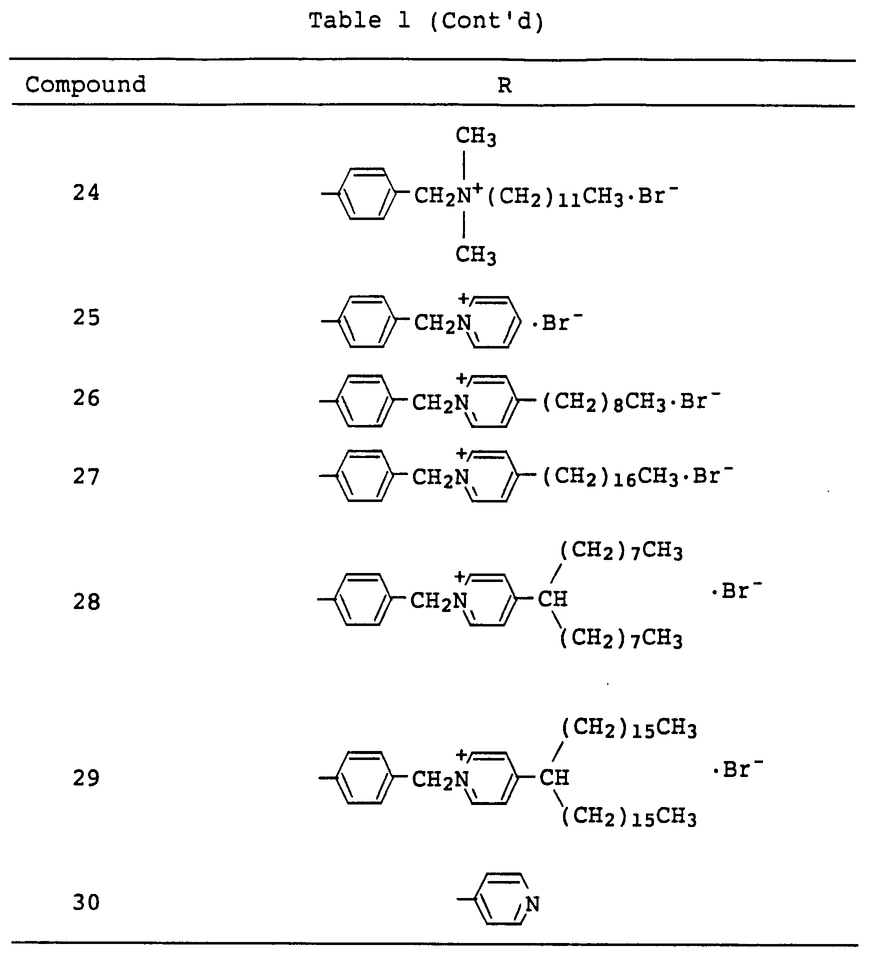

In Table 1, Compound Nos. 3, 4, 6-10, 12, 14, 18-20 and 23-24 are novel compounds.

The terpyridine derivative of the present invention represented by formula (I) are administered orally or parenterally.

A daily dose of the terpyridine derivative of the present invention is preferably in the range of 1 mg to 1000 mg for adult.

For oral administration, the terpyridine derivative is mixed with an excipient, a degrading agent, a binder, a lubricant, an antioxidant, a coating agent, a coloring agent, a corrigent, a surfactant, a plasticizer, etc. to prepare into granules, powders, capsules or tablets; these pharmaceutical preparations are orally administered. In the case of parenteral administration, the terpyridine derivative of the present invention can be administered in the form of an injection, a drip or a suppository.

These pharmaceutical preparations may be prepared in a conventional manner for making pharmaceuticals.

Examples of the excipient include mannitol, xylitol, sorbitol, glucose, refined sugar, lactose, crystalline cellulose, sodium carboxylmethyl cellulose, calcium hydrogenphosphate, wheat starch, rice starch, corn starch, potato starch, sodium carboxymethyl starch, dextrin, α-cyclodextrin, β-cyclodextrin, carboxylvinyl polymer, light silicic anhydride, titanium oxide, magnesium metasilicate aluminate, polyethylene glycol, medium chain fatty acid triglyceride, etc.

Examples of the degrading agent include hydroxypropyl cellulose having a low degree of substitution, carboxymethyl cellulose, calcium carboxymethyl cellulose, sodium carboxymethyl cellulose, Sodium Crosscarmellose type A (Ac-Di-Sol), starch, crystalline cellulose, hydroxypropyl starch and partially alphatized starch.

Examples of the binder include methyl cellulose, hydroxypropyl cellulose, hydroxypropylmethyl cellulose, polyvinylpyrrolidone, gelatin, gum arabic, ethyl cellulose, polyvinyl alcohol, prulan, alphatized starch, agar, tragacanth, sodium alginate and sodium alginate propylene glycol ester.

Examples of the lubricant include stearic acid, magnesium stearate, calcium stearate, polyoxyl stearate, cetanol, talc, hardened oil, sucrose fatty acid esters, dimethylpolysiloxane, microcrystalline wax, bees wax and haze wax.

Examples of the antioxidant include dibutylhydroxytoluene (BHT), propyl gallate, butylhydroxyanisole (BHA), α-tocopherol and citric acid.

Examples of the coating agent include hydroxypropylmethyl cellulose, hydroxypropyl cellulose, methyl cellulose, ethyl cellulose, hydroxypropylmethyl cellulose phthalate, hydroxypropylmethyl cellulose acetate succinate, carboxymethylethyl cellulose, cellulose acetate phthalate, polyvinyl acetal diethylaminoacetate, aminoalkyl methacrylate copolymers, hydroxypropylmethyl cellulose acetate succinate, methacrylic acid copolymer, cellulose acetate trimellitate (CAT), polyvinyl acetate phthalate and shellac.

Examples of the coloring agent include tar pigment and titanium oxide.

Examples of the corrigent include citric acid, adipic acid, ascorbic acid and menthol.

Examples of the surfactant include polyoxyethylene-hardened castor oil, glycerine monostearate, sorbitan monostearate, sorbitan monopalmitate, sorbitan monolaurate, polyoxyethylene- polyoxypropylene block copolymer, polysorbates, sodium laurylsulfate, macrogols and sucrose fatty acid esters.

Examples of the plasticizer include triethyl citrate, triacetyne and cetanol.

Hereinafter the present invention is described in more detail, by referring to Preparation Examples, Examples and Test Examples.

### Preparation Examples

### Reference Example 1

### 1-[2-Oxo-2-(2-pyridyl)ethyl]pyridinium iodide

While stirring, a mixture of 55.16 g of 2-acetylpyridine, 150 ml of pyridine and 120 g of iodine was gradually heated to 100°C over 20 minutes and then at 100°C for further 30 minutes. After cooling to room temperature, the reaction mixture was thoroughly washed with benzene and crystallized from ethanol-water to give 130.78 g of the title compound.
m.p. 198-199°C (decompd.)

### Reference Example 2

### (E)-3-(4-methylphenyl)-1-(2-pyridyl)-2-propen-1-one

While stirring 10 ml of 3% sodium hydroxide-methanol solution was dropwise added at 0°C to a mixture of 5.61 ml of 2-acetylpyridine, 5.90 ml of p-tolualdehyde and 20 ml of methanol. The mixture was stirred at 0°C for further 4 hours. After completion of the reaction, the solvent was distilled off under reduced pressure. The residue was crystallized from methanol to give 7.45 g of the title compound.

2-Acetylpyridine was reacted with the corresponding aldehyde in substantially the same procedures as in Reference Example 2 to give the following compounds.
(E)-3-Phenyl-1-(2-pyridyl)-2-propen-1-one
(E)-3-(2-Methylphenyl)-1-(2-pyridyl)-2-propen-1-one
(E)-3-(4-Ethylphenyl)-1-(2-pyridyl)-2-propen-1-one
(E)-3-(2,5-Dimethylphenyl)-1-(2-pyridyl)-2-propen-1-one
(E)-3-(4-Chlorophenyl)-1-(2-pyridyl)-2-propen-1-one
(E)-3-(2-Hydroxyphenyl)-1-(2-pyridyl)-2-propen-1-one
(E)-3-(4-Methoxyphenyl)-1-(2-pyridyl)-2-propen-1-one
(E)-3-(3-Nitrophenyl)-1-(2-pyridyl)-2-propen-1-one
(E)-1-(2-Pyridyl)-3-(4-pyridyl)-2-propen-1-one

### Reference Example 3

### (E)-3-[4-(2-hydroxyethoxy)phenyl]-1-(2-pyridyl)-2-propen-1-one

While stirring 50 ml of 3% potassium hydroxide-methanol solution was dropwise added at 0°C to a mixture of 12.3 ml of 2-acetylpyridine, 16.6 g of 4-(2-hydroxyethoxy)benzaldehyde and 20 ml of methanol. The mixture was stirred at 0°C for further 4 hours. After completion of the reaction, the solvent was distilled off under reduced pressure. The residue was purified by silica gel column chromatography (developing solvent: benzene : ethanol = 3 :1) to give 25 g of the title compound as oil.

2-Acetylpyridine was reacted with the corresponding aldehyde in substantially the same procedures as in Reference Example 3 to give the following compounds.
(E)-3-(3-Methylphenyl)-1-(2-pyridyl)-2-propen-1-one
(E)-3-(4-Isopropylphenyl)-1-(2-pyridyl)-2-propen-1-one
(E)-3-(2,4-Dimethylphenyl)-1-(2-pyridyl)-2-propen-1-one
(E)-3-(4-Ethoxyphenyl)-1-(2-pyridyl)-2-propen-1-one

### Reference Example 4

### 4-Heptadecylpyridine

While stirring under cooling with dry ice, 0.83 g of sodium, then 0.40 g of iron (III) nitrate nonahydrate and finally 9.00 g of sodium were added over an hour to 800 ml of ammonia liquefied by cooling with dry ice. After stirring for 2 hours under cooling, 40.7 ml of γ-picoline was added to the mixture over 15 minutes. After the mixture was stirred for further 30 minutes under cooling, a solution mixture of 128.2 ml of cetyl bromide and 100 ml of toluene was dropwise added over an hour. After stirring at room temperature for 12 hours, water was poured onto the mixture followed by extraction with ether. Hydrogen chloride gas was blown into the ethereal solution. The resulting precipitates were filtered by suction, washed with ethyl acetate and dried. An aqueous sodium hydroxide solution was added to the precipitates to render alkaline, followed by extraction with ether. The solvent was distilled off under reduced pressure and the residue was distilled in vacuum to give 94.8 g of the title compound.

The following compound was obtained in substantially the same manner as in Reference Example 4 except for using octyl bromide instead of cetyl bromide.
4-Nonylpyridine

### Reference Example 5

### 4-(1-Octylnonyl)pyridine

While stirring under cooling with dry ice, 0.40 g of sodium, then 0.20 g of iron (III) nitrate nonahydrate and finally 5.00 g of sodium were added over 40 minutes to 300 ml of ammonia liquefied by cooling with dry ice. After stirring for an hour and a half under cooling, 48.1 g of 4-nonylpyridine obtained in Reference Example 4 was added to the mixture over 30 minutes. After the mixture was stirred for further 2 hours under cooling, 40.5 ml of octyl bromide was added to the reaction mixture over 30 minutes. After stirring at room temperature for 12 hours, water was poured onto the mixture followed by extraction with ether. Hydrogen chloride gas was blown into the ethereal solution. The resulting precipitates were filtered by suction and dried. An aqueous sodium hydroxide solution was added to the precipitates to render alkaline, followed by extraction with ether. The solvent was distilled off under reduced pressure and the residue was distilled in vacuum to give 14.4 g of the title compound.

### Reference Example 6

### 4-(1-Hexadecylheptadecyl)pyridine

While cooling with dry ice under stirring, To 600 ml of ammonia liquefied by cooling with dry ice were added, 0.40 g of sodium, then 0.30 g of iron (III) nitrate nonahydrate and finally 6.50 g of sodium were added over an hour. After stirring for 2 hours under cooling, a solution mixture of 94.8 g of 4-heptadecylpyridine obtained in Reference Example 4 and 300 ml of dehydrated ether was added to the mixture over 80 minutes. After stirring at room temperature for 6 hours, ammonia was distilled off. Then 100 ml of ether was added to the residue and 91.1 ml of cetyl bromide was added to the resulting mixture over 30 minutes. After stirring at room temperature for 12 hours, water was poured onto the mixture followed by extraction with ether. Hydrogen chloride gas was blown into the ethereal solution. The resulting precipitates were filtered by suction and dried. An aqueous sodium hydroxide solution was added to the precipitates to render alkaline, followed by extraction with ether. The solvent was distilled off under reduced pressure and unreacted starting materials were removed by distillation in vacuum. The residue was recrystallized from ethanol to give 29.1 g of the title compound.

### Preparation Example 1

### 4'-(4-Methylphenyl)-2,2':6',2''-terpyridine (Compound 5)

To 9.28 g of 1-[2-oxo-2-(2-pyridyl)ethyl]pyridinium=iodide obtained in Reference Example 1, 6.35 g of (E)-3-(4-methylphenyl)-1-(2-pyridyl)-2-propen-1-one obtained in Reference Example 2 and 22 g of ammonium acetate was added 50 ml of methanol. The mixture was refluxed for 8 hours. After cooling, the product was taken out by filtration and recrystallized from acetone to give 5.31 g of the title compound.
m.p. 166-167°C
¹H NMR (CDCl₃) δ (ppm): 2.43 (3H, s), 7.29-7.38 (4H, m), 7.80-7.92 (4H, m), 8.65-8.75 (6H, m)
MS (EI) 323 (M⁺)
The following compounds were prepared by substantially the same manner as in Preparation Example 1 except that the corresponding α,β-unsaturated ketone prepared in a manner similar to Reference Example 2 or 3 was used instead of (E)-3-(4-methylphenyl)-1-(2-pyridyl)-2-propen-1-one.
4'-Phenyl-2,2':6',2''-terpyridine (Compound 2)
m.p. 205-206°C
4'-(2-Methylphenyl)-2,2':6',2''-terpyridine (Compound 3)
m.p. 169-170°C
4'-(3-Methylphenyl)-2,2':6',2''-terpyridine (Compound 4)
m.p. 126-128°C
4'-(4-Ethylphenyl)-2,2':6',2''-terpyridine (Compound 7)
m.p. 113-114°C
4'-(4-Isopropylphenyl)-2,2':6',2''-terpyridine (Compound 8)
m.p. 150-152°C
4'-(2,4-Dimethylphenyl)-2,2':6',2''-terpyridine (Compound 9)
m.p. 127-128°C
4'-(2,5-Dimethylphenyl)-2,2':6',2''-terpyridine (Compound 10)
m.p. 139-140°C
4'-(4-Chlorophenyl)-2,2':6',2''-terpyridine (Compound 11)
m.p. 172-173°C
2-(2,2':6',2''-Terpyridin-4'-yl)phenol (Compound 12)
m.p. 266-268°C
4'-(4-Methoxyphenyl)-2,2':6',2''-terpyridine (Compound 13)
m.p. 150-151°C
4'-(4-Ethoxyphenyl)-2,2':6',2''-terpyridine (Compound 14)
m.p. 172-173°C
4'-(3-Nitrophenyl)-2,2':6',2''-terpyridine (Compound 17)
m.p. 184-185°C
4'-(4-Pyridyl)-2,2':6',2''-terpyridine (Compound 30)
m.p. 225-226°C

### Preparation Example 2

### 2-[4-(2,2':6',2''-Terpyridin-4'-yl)phenoxy]ethyl acetate (Compound 16)

To 19.6 g of 1-[2-oxo-2-(2-pyridyl)ethyl]pyridinium iodide obtained in Reference Example 1, 16.2 g of (E)-3-(4-(2-hydroxyethoxy)phenyl]-1-(2-pyridyl)-2-propen-1-one obtained in Reference Example 3 and 46 g of ammonium acetate was added 130 ml of acetic acid. The mixture was then refluxed for 8 hours. After the reaction was completed, the solvent was distilled off under reduced pressure and chloroform was added to the residue. After washing successively with water, 5% aqueous potassium hydroxide solution and water, the mixture was dried over anhydrous magnesium sulfate. The solvent was distilled off under the reduced pressure and the residue was purified by alumina column chromatography (developing solvent: chloroform) to give 6.4 g of the title compound.
m.p. 162-163°C

### Preparation Example 3

### 2-[4-(2,2':6',2''-Terpyridin-4'-yl)phenoxy]ethanol (Compound 15)

After 5.0 g of 2-[4-(2,2':6',2''-terpyridin-4'-yl)phenoxy]ethyl acetate obtained in Reference Example 2 was dissolved in 300 ml of hot methanol, 5 ml of 50% aqueous potassium hydroxide solution was added to the solution with stirring. The crystals obtained a few minutes later were taken out by filtration, washed with cold methanol and then recrystallized from ethanol to give 4.3 g of the title compound.
m.p. 198-199°C

### Preparation Example 4

### 4'-(4-Methylphenyl)-2,2':6',2''-terpyridin trihydrochloride (Compound 6)

To 1.85 g of 4'-(4-methylphenyl)-2,2':6',2''-terpyridine obtained in Reference Example 1 was added 70 ml of ethyl acetate. The mixture was heated to dissolve. While stirring, 20 ml of 4 N hydrochloric acid-ethyl acetate solution was added to the thus obtained solution. After stirring at room temperature for 30 minutes, the crystals obtained were filtered by suction to give 2.38 g of the title compound.
m.p. 244-246°C

### Preparation Example 5

### 4'-(3-Aminophenyl)-2,2':6',2''-terpyridine (Compound 18)

While stirring, 2.0 g of activated Raney nickel was slowly added at 30 to 40°C to a mixture of 5.0 g of 4'-(3-aminophenyl)-2,2':6',2''-terpyridine obtained in Preparation Example 1, 1.7 ml of hydrazine monohydrate and 150 ml of ethanol. The mixture was refluxed for 2 hours. After completion of the reaction, the reaction mixture was spontaneously filtered to remove the catalyst, while the mixture was hot. The filtrate was concentrated. The crystals obtained were filtered, purified by alumina column chromatography (developing solvent: chloroform) and then recrystallized from acetone to give 2.79 g of the title compound.
m.p. 218-220°C

### Preparation Example 6

### 4-(2,2':6',2''-Terpyridin-4'-yl)benzoic acid (Compound 19)

After 1.32 g of potassium permanganate was added to a mixture of 1.13 g of 4'-(4-methylphenyl)-2,2':6',2''-terpyridine obtained in Preparation Example 1, 9 ml of pyridine and 5 ml of water, the mixture was refluxed for 3 hours. Sodium hydrogensulfite was added to the reaction mixture to decompose unreacted potassium permanganate. Manganese dioxide was removed by suction with heating. The solvent was removed by distillation under reduced pressure and the residue was dissolved in 40 ml of ethanol and 10 ml of water with heating. To the solution was added 0.70 ml of 12 N hydrochloric acid aqueous solution. The crystals obtained were filtered by suction and thoroughly washed with water. The crude product was dissolved in a mixture of 10 ml of 1 N sodium hydroxide aqueous solution, 20 ml of water and 30 ml of ethanol. Insoluble matters were removed by spontaneous filtration. After 0.83 ml of 12 N hydrochloric acid aqueous solution was added to the filtrate, the crystals obtained were taken out by suction filtration. After washing successively with water and methanol, the crystals were dried in vacuum to give 0.68 g of the title compound.
m.p. > 300°C
¹H NMR (CDCl₃) δ (ppm): 7.30-7.40 (4H, m), 7.82-7.91 (4H, m), 8.66-8.76 (6H, m)
MS (EI) 353 (M⁺)

### Preparation Example 7

### 2-(2,2':6',2''-Terpyridin-4'-yl)terephthalate dihydrate (Compound 20)

After 2.29 g of potassium permanganate was added to a mixture of 1.01 g of 4'-(2,5-dimethylphenyl)-2,2':6',2''-terpyridine obtained in Preparation Example 1, 9 ml of pyridine and 5 ml of water, the mixture was refluxed for 3 hours. After the reaction was completed, manganese dioxide was removed by suction with heating. The solvent was removed by distillation under reduced pressure and 80 ml of water was added to the residue to dissolve. The mixture was neutralized by adding 2 N hydrochloric acid aqueous solution. The crystals obtained were filtered by suction and thoroughly washed with water. Recrystallization from ethanol gave 0.54 g of the title compound.
m.p. 258-260°C

### Preparation Example 8

### 4'-(4-Bromomethylphenyl)-2,2':6',2''-terpyridine (Compound 21)

After 0.060 g of benzoyl peroxide was added to a mixture of 2.00 g of 4'-(4-methylphenyl)-2,2':6',2''-terpyridine obtained in Preparation Example 1, 1.20 g of N-bromosuccinimide and 30 ml of carbon tetrachloride, the mixture was refluxed for 3 hours. After the reaction was completed, the solvent was removed by distillation under reduced pressure. After washing with water, the residue was recrystallized from chloroform-methanol to give 2.14 g of the title compound.
m.p. 161-162°C

### Preparation Example 9

### 4-(1-Hexadecylheptadecyl)-1-[4-(2,2':6',2''-terpyridin-4'-yl)phenylmethyl]pyridinium bromide (Compound 29)

A mixture of 1.45 g of 4'-(4-bromomethylphenyl)-2,2':6',2''-terpyridine obtained in Preparation Example 8, 1.95 g of 4-(1-hexadecylheptadecyl)pyridine obtained in Reference Example 6 and 30 ml of ethanol was refluxed for 23 hours. The solvent was removed by distillation under reduced pressure. After washing with ethyl acetate, the residue was purified by alumina column chromatography (developing solvent: chloroform) and then recrystallized from methanol to give 2.14 g of the title compound.
m.p. 113-114°C
The following compounds were obtained by substantially the same manner as in Preparation Example 9 except that the corresponding amine was used instead of 4-(1-hexadecylheptadecyl)pyridine.
1-[4-(2,2':6',2''-Terpyridin-4'-yl)phenylmethyl]pyridinium bromide (Compound 25)
m.p. 238-240°C (decompd.)
4-Nonyl-1-[4-(2,2':6',2''-terpyridin-4'-yl)phenylmethyl]pyridinium bromide (Compound 26)
m.p. 241-243°C (decompd.)
4-Heptadecyl-1-[4-(2,2':6',2''-terpyridin-4'-yl)phenylmethyl]pyridinium bromide (Compound 27)
m.p. 96-98°C
4-(1-Octylnonyl)-1-[4-(2,2':6',2''-terpyridin-4'-yl)phenylmethyl]pyridinium bromide (Compound 28)
m.p. 118-119°C
N,N,N-Trimethyl-N-[4-(2,2':6',2''-terpyridin-4'-yl)phenylmethyl]ammonium bromide (Compound 23)
m.p. 265-268°C (decompd.)
N-Dodecyl-N,N-dimethyl-N-[4-(2,2':6',2''-terpyridin-4'-yl)phenylmethyl]ammonium bromide (Compound 24)
m.p. 157-158°C
4'-[4-(1-pyrrorylmethyl)phenyl]-2,2':6',2''-terpyridine (Compound 22)
m.p. 153-154°C

### Example 1. Tablet

| Composition, per tablet | |
|---|---|
| Compound 6 | 30 mg |
| Potato starch | 20 mg |
| Crystalline cellulose | 20 mg |
| Carboxymethyl cellulose | 20 mg |
| Hydroxypropyl cellulose | 9 mg |
| Magnesium stearate | 1 mg |

### Method

After 200 g of Compound 6, 200 g of potato starch, 200 g of crystalline cellulose, 200 g of carboxymethyl cellulose and 90 g of hydroxypropyl cellulose are mixed with each other, the mixture is ground into powders with a grinder. The powders are put in a stirring granulator. A sufficient quantity of water is added to the powders for granulation. The granules are then dried with a drier on a fluidized bed. To the granules is added 10 g of magnesium stearate. Using a tableting machine, the mixture is prepared into a tablet containing 30 mg of Compound 6 (100 mg, 6 mm in diameter).

### Example 2. Granule

| Composition, per granule | |
|---|---|
| Compound 5 | 100 mg |
| Refined sugar | 200 mg |
| Mannitol | 200 mg |
| Corn starch | 470 mg |
| Hydroxypropyl cellulose | 20 mg |
| Polysorbate 80 | 10 mg |

### Method

After 100 g of Compound 5 is ground in a ball mill, 200 g of refined sugar and 470 g of corn starch are added to the powders. After blending, a solution of 20 g of hydroxypropyl cellulose and 10 g of polysorbate 80 in 250 g of a mixture of purified water and ethanol (1 : 2) is added to the mixture, followed by mixing them with a mixer. The mixture is extruded through a screen having an orifice of 0.5 mm and then granulated with a granulator. The granules are dried with a dryer on a fluidized bed to obtain a granule containing 10% of Compound 5.

### Example 3. Fine granule

| Composition, per chartula | |
|---|---|
| Compound 30 | 100 mg |
| Lactose | 300 mg |
| Glucose | 230 mg |
| Corn starch | 330 mg |
| Hydroxypropyl cellulose | 30 mg |
| Polysorbate 80 | 10 mg |

### Method

After 100 g of Compound 30 is ground into powders with a jet grinder, 300 g of lactose, 230 g of glucose and 330 g of corn starch are added to the powders. After mixing, 30 g of hydroxypropyl cellulose and 10 g of polysorbate 80 are dissolved in 800 g of water. Using the solution as a binder, the mixture is prepared into fine granules with a grinder on a fluidized bed. The granules are dried to obtain a fine granule containing 10% of Compound 30.

### Example 4. Powders

| Composition, per chartula | |
|---|---|
| Compound 29 | 200 mg |
| Lactose | 800 mg |

### Method

By uniformly blending 200 g of Compound 29 with 800 g of lactose, powders are prepared to obtain powders containing 20% of Compound 29.

### Example 5. Capsule

| Composition, per capsule | |
|---|---|
| Compound 6 | 50.0 mg |
| Lactose | 50.0 mg |
| Mannitol | 145.0 mg |
| Hydroxypropyl cellulose having a low substitution degree | 15.0 mg |
| Hydroxypropylmethyl cellulose | 12.5 mg |
| Polysorbate 80 | 10.0 mg |
| Calcium stearate | 7.5 mg |
| Hardened oil | 10.0 mg |

### Method

After 100 g of Compound 6 is ground into powders in a ball mill, 100 g of lactose, 290 g of mannitol and 30 g of hydroxypropyl cellulose having a low substitution degree are added to the powders. After blending, a solution of 25 g of hydroxypropyl cellulose and 20 g of polysorbate 80 in 800 g of a mixture of purified water and ethanol (1 : 2) is added to the mixture as a binder. The mixture is granulated with a granulator on a fluidized bed, dried and sieved with a No. 30 sieve. To the thus sieved granules are added 15 g of calcium stearate and 20 g of hardened oil. The mixture is filled in No. 1 capsule in a dose of 300 mg. Thus, a capsule containing 50 mg of Compound 6 is obtained.

### Example 6. Injection

| Composition, per ampoule | |
|---|---|
| Compound 6 | 10 mg |
| Distilled water | 2 ml in total |

### Method

Distilled water for injection is added to 10 g of Compound 6 to dissolve and make the total volume 2000 ml. The solution is dispensed in a 2 ml ampoule in a conventional manner. An ampoule containing 10 mg of Compound 6 (0.5%, 2 ml) is thus obtained.

Hereinafter the present invention is described below in more detail with reference to test examples.

### Test Example 1. Synthesis-stimulating activity on NGF

The activity of accelerating secretion of NGF was evaluated by the following method.

### Compounds

Each compound shown in Table 1 was dissolved in dimethylsulfoxide (DMSO) to adjust the concentration in the range of 2 mg/ml to 10 mg/ml.

### Cell

Astroglial cells derived from the mouse forebrain (cells that synthesize and secrete NGF)

### Method

Astroglial cells obtained from the mouse forebrain were cultured into 8 x 10⁵ cells/ml in Dulbecco's modified Eagle's medium obtained from Gibco, glucose-rich, containing 20% fetal calf serum, 100 units/ml of penicillin and 100 µg/ml of streptomycin. A 0.1 ml/well aliquot was inoculated on a 96-well plate obtained from Falcon and having an area of 0.32 cm² per well and cultured at 37°C in an atmosphere of 5% CO₂. The culture medium was removed 72 hours later and instead, a 0.1 ml/well aliquot of fresh Dulbecco's modified Eagle's medium containing 0.5% bovine albumin powders obtained from Armour, 100 units/ml of penicillin and 100 µg/ml of streptomycin was supplemented to the system followed by culturing at 37°C in 5% CO₂. The above medium containing the compound each having various concentrations was exchanged 72 hours later. Herein each compound was dissolved in DMSO and added so as to have the final concentration as indicated in Table 2 below. For the purpose of comparison, the medium which contains only DMSO was also prepared (control).

After culturing for 24 hours in these various medium, the concentration of NGF in each medium was determined by enzyme immunoassay, see Furukawa et al., J. Neurochem., 40, 734 (1983).

The synthesis-stimulating activity on NGF by the compound of the present invention is shown in terms of a ratio when the NGF level in the control containing only DMSO was made 1.0.

### Results

The results were shown in Table 2.

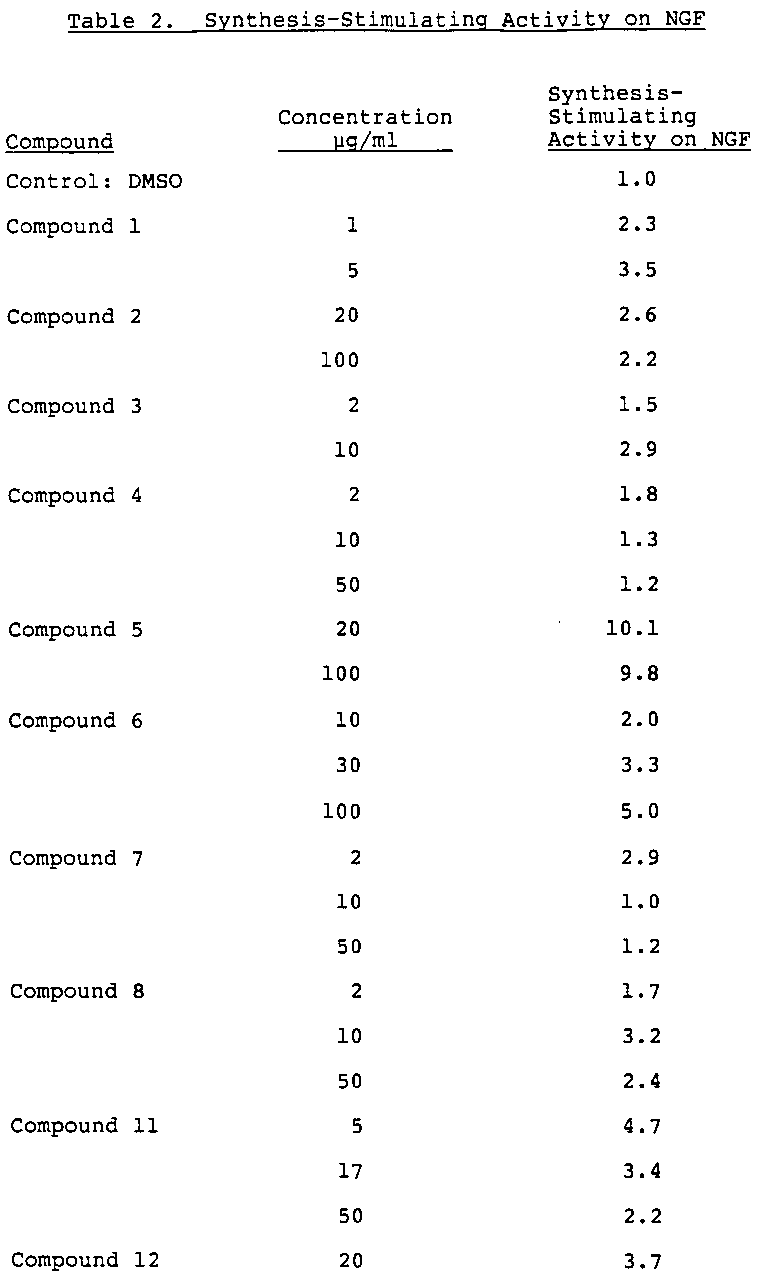

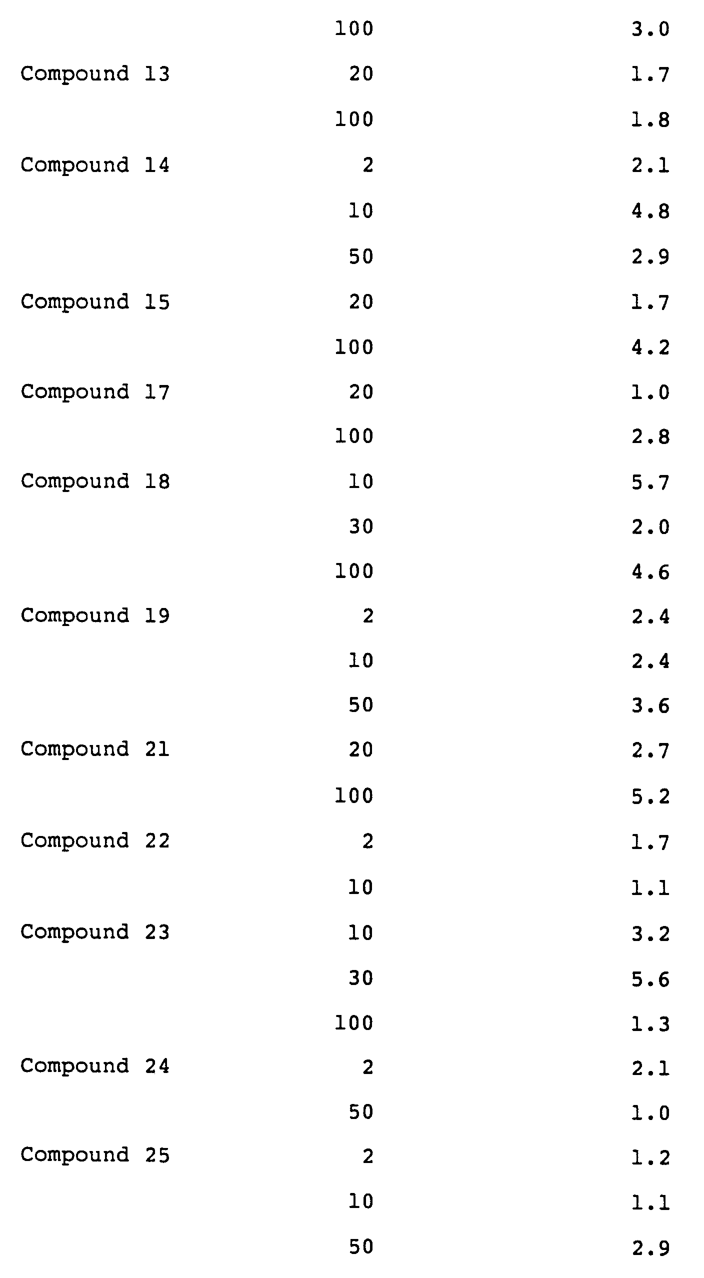

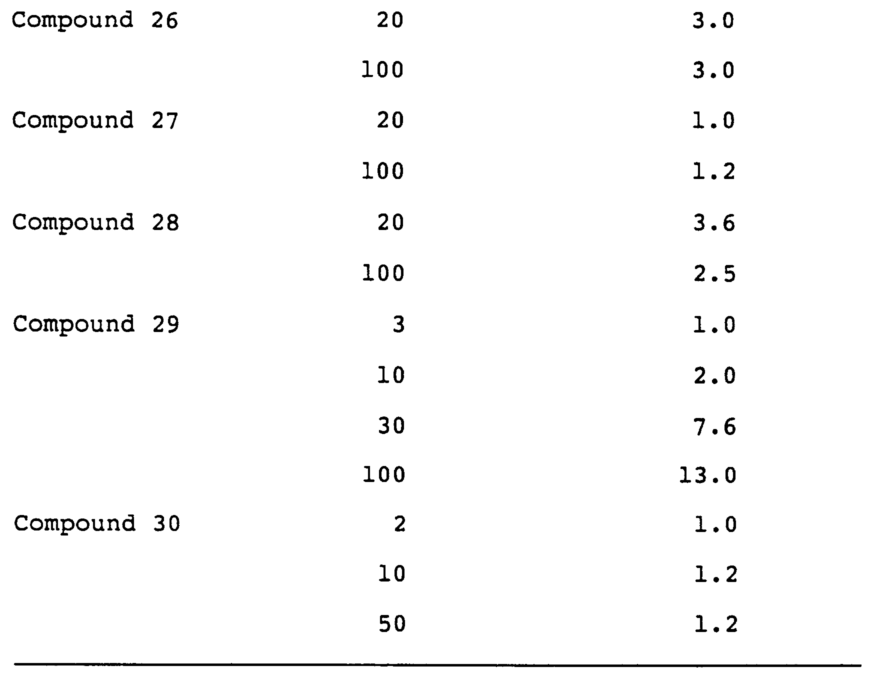

### Test Example 2. Test on neurotrophic activity

The neurotrophic activity was determined by the following method.

### Compounds

Each compound was dissolved in DMSO to adjust the concentration in the range of 2 mg/ml to 10 mg/ml.

### Cell

Cerebral cortical neuronal cells from an 18-day-old rat embryo

### Method

Neuronal cells in a 1 : 1 mixture of Dulbecco's modified Eagle's minimum essential medium, obtained from Gibco, and Ham's F12 medium, obtained from Gibco, containing 20% fetal bovine serum were plated by a 0.5 ml/well aliquot on 24-well dishes coated with polyethylenimine (an area of 2 cm² per well, obtained from Corning) at a density of 3.2 x 10⁶ cells/ml and incubated at 37°C in 5% CO₂. After 24 hours of incubation, the culture medium was removed and a 0.5 ml/well aliquot of a serum-free fresh DF medium containing the respective compounds having various concentrations, 5 µg/ml of transferrin, 5 µg/ml of insulin and 20 pmoles/ml of progesterone was supplemented to the system. Each compounds of the present invention was dissolved in DMSO to have the final concentration as indicated in Table 3 below. For the purpose of comparison, the medium containing only DMSO was added was also prepared.

At this stage, the two same plates were used. After 72 hours of incubation in various medium, hypoxia was loaded on one plate by reducing the concentration of oxygen to the minimum concentration of 1% using a N₂-O₂-CO₂ incubator (manufactured by Tabai, Model BNP-100) and culturing for 4 hours under the conditions. Another plate was cultured in an atmosphere of 5% CO₂ and 95% air, without hypoxic load. After the incubation was continued for further 48 hours, living cells were counted. The culture medium was removed and fluorescein diacetate (FDA) reagent was reacted. After washing with phosphate buffer, pH 7.4, the fluorescent intensity (E x 485/Em 530) was measured by CytoFlour^{TM} 2300, manufactured by Millipore.

The neurotrophic activity of the compounds of the present invention is shown in terms of a ratio when the fluorescent intensity of the control containing only DMSO was made 1.0.

### Results

The results were shown in Table 3.

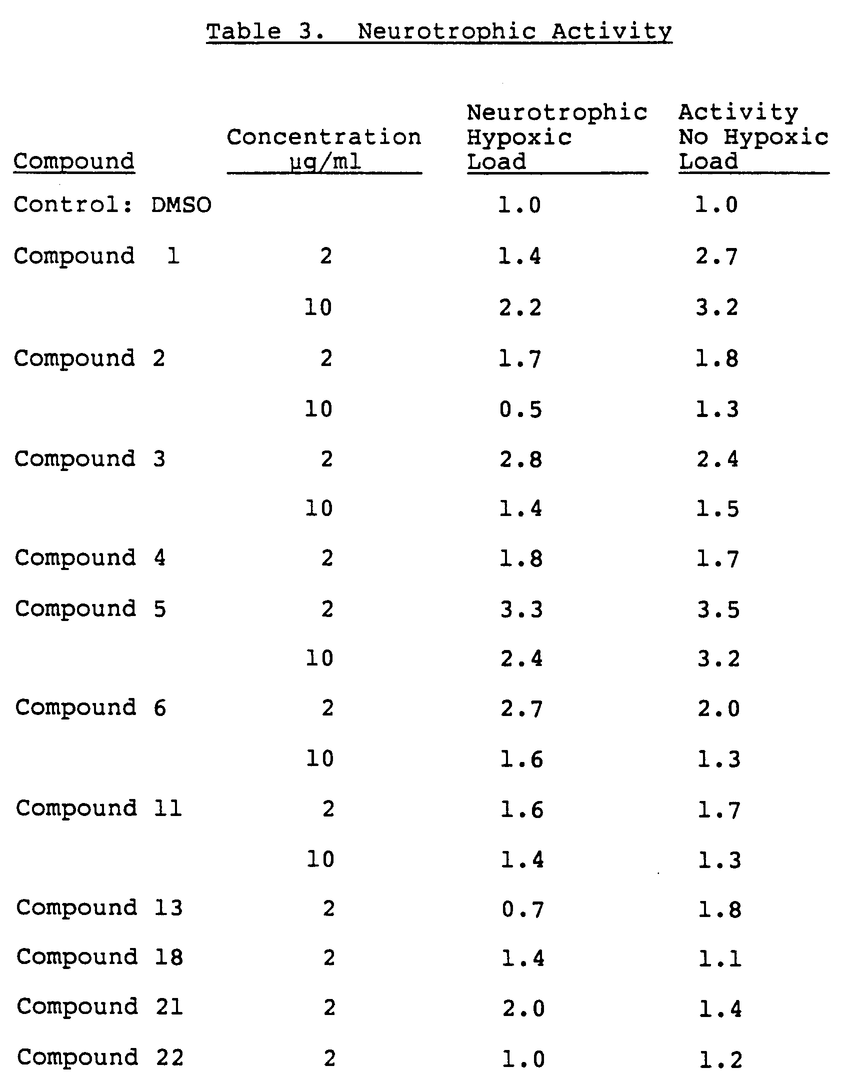

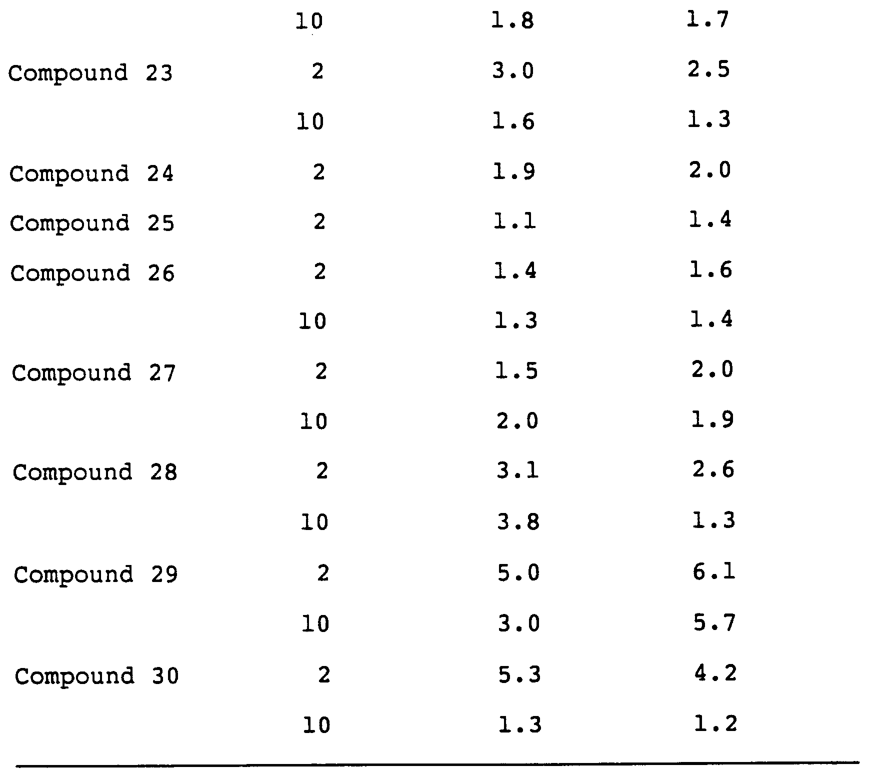

### Test Example 3. Test on delayed neuronal death induced by cerebral ischemia in Mongolian gerbils

Neuronal damages induced by cerebral ischemia was evaluated in the both common carotid artery-occlusion model Mongolian gerbils.

### Animals

Male Monglian gerbils, supplied by Shin Nihon Dobutsu Co., Ltd., Saitama, Japan and weighing 60 to 90 g.

### Method

The gerbils were anesthetized lightly with ether and placed in the supine position. After local infiltration of xylocaine, both common carotid arteries were exposed through a ventral midline incision. The arteries were then clamped with aneurysm clips for 3 minutes. Thereafter the clips were removed and the skin was sutured. Sham operated animals were treated in the same manner, except that clamping was not done. The animals were anesthetized with ether 7 days after the bilateral common carotid arteries had been occluded for 3 minutes, and the brains were perfused with a 10% buffered formalin solution given through the left cardiac ventricle. The hippocampal region was cut coronally into 3- to 4-mm thick slices, embedded in paraffin and processed using the step section technique. The preparations were stained with hematoxylin and eosin. Ischemic neuronal damage was graded on 4 levels from scores 0 to 3: score 0 (-), normal neurons; score 1 (+), a few neurons damaged (as few as one neuron damaged); score 2 (++), many neurons damaged; and score 3 (+++), majority of neurons damaged.

### Compounds

Compounds to be tested were suspended in gum arabic or dissolved in physiological saline, which was intraperitoneally given at determined times in doses of 10 mg/kg to 100 mg/kg, before and after the induced ischemia.

### Results

In the sham operated gerbils, CAl neurons in the hippocampus were not damaged at all, whereas at 7 days after the 3 minute bilateral common carotid artery occlusion in the gerbils, the CAl neurons in the hippocampus were found to be obviously damaged, by an light microscopic examination. Destruction and disappearance of the hippocampal CAl neurons were prevented when the compounds of the present invention were given intraperitoneally. In particular, Compound 6 prevented damages of the hippocampal CAl neurons even when intraperitoneally given 30 minutes after or an hour after the ischemia had been induced. The results are shown in Table 4 below.

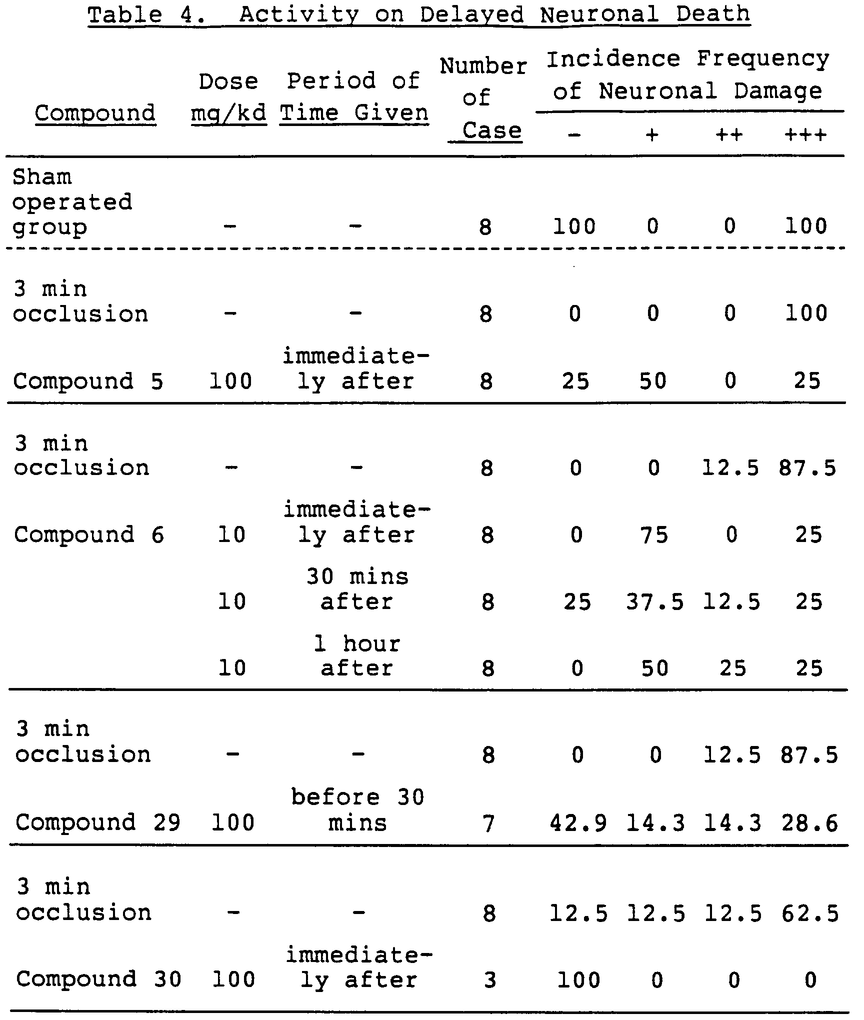

### Industrial Utilization

NGF plays a role for maintaining the survival and function of certain nerve cells and exhibits the activity of modification, repair and protection of the nerve cells. The terpyridine derivatives of the present invention exert both or one of the NGF synthesis-stimulating activity and the activity of prolonging the life of the cultured cerebral cortical neuronal cells. It is thus expected that the terpyridine derivatives of the present invention would act on nerve cells directly or indirectly to show the effects of improving and treating neural disorders associated with denaturation of neurons. The terpyridine derivatives of the present invention are expected to be effective for improving and treating conditions or diseases associated with traumatic, chemical (alcohol or carcinostatic agents), inflammatory, metabolic (observed in diabetes, etc.) or idiopathic denaturation of peripheral nerves. The terpyridine derivatives of the present invention may also be used as drugs for the improvement and treatment of psychological malfunction, locomotor malfunction or paravertebral anesthesia, etc., caused by conditions or diseases associated with denaturation of central nerves, e.g., Alzheimer and cerebro-vascular dementia, Down's syndrome, Parkinson's disease, Huntington's chorea, cerebral ischemia, cerebral infarction, head trauma, and the like.

## Claims

1. A pharmaceutical composition comprising as an effective ingredient a terpyridine derivative represented by formula (I): 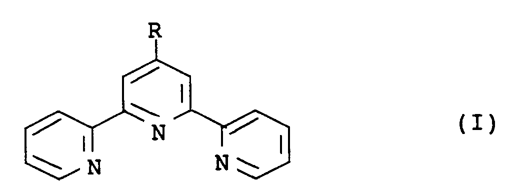 wherein R represents a hydrogen atom; an unsubstituted phenyl group; a phenyl group substituted with at least one substituent selected from the group consisting of an alkyl group, an alkoxy group, hydroxy group, nitro group, amino group, carboxy group, bromomethyl group, 2-hydroxyethoxy group, 2-acetoxyethoxy group and a halogen atom; pyridyl group; or a functional group represented by any one of (II)-1 through (II)-8 shown below: 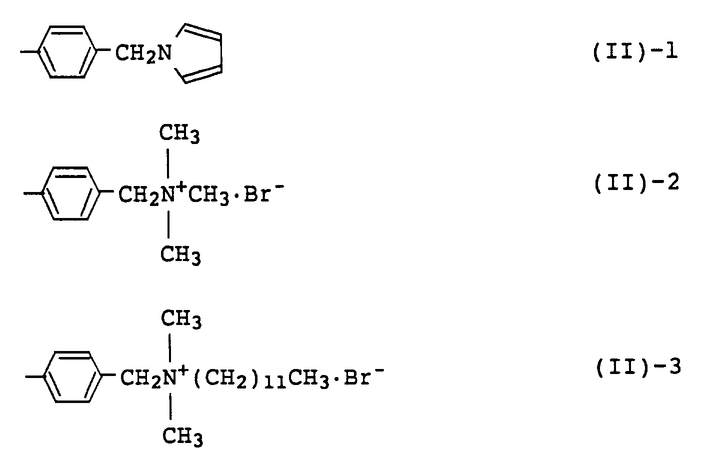 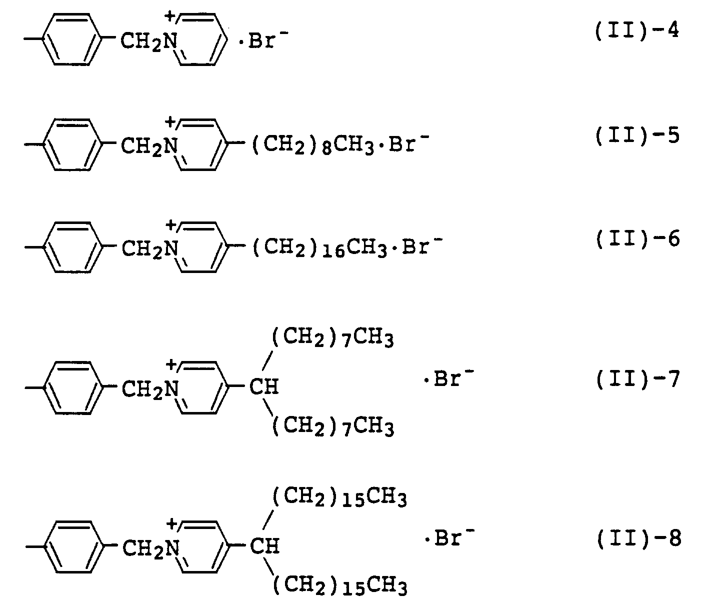 or a pharmaceutically acceptable salt thereof.

2. A pharmaceutical composition according to claim 1, which is for improving or treating diseases caused by denaturation of neurons.

3. A terpyridine derivative or a pharmaceutically acceptable salt thereof according to claim 1, for use as an effective ingredient of a pharmaceutical composition.

4. A terpyridine derivative or a pharmaceutically acceptable salt thereof according to claim 3, which is a pharmaceutical composition for improving or treating diseases caused by denaturation of neurons.

5. Use of a terpyridine derivative or a pharmaceutically acceptable salt thereof according to claim 1, in the preparation of a pharmaceutical composition for improving or treating diseases caused by denaturation of neurons.

6. A method for improving or treating diseases caused by denaturation of neurons which comprises administering to human an effective dose of a terpyridine derivative or a pharmaceutically acceptable salt thereof according to claim 1.
